# EUROPÄISCHE PATENTSCHRIFT

(11) **EP 0 405 298 B1**
(45) Veröffentlichungstag und Bekanntmachung des Hinweises auf die Patenterteilung: **11.05.1994**
(21) Anmeldenummer: 90111529.5
(22) Anmeldetag: 19.06.1990
(51) Int. Cl.: C07D 495/04, A61K 31/38

(54) **Thienopyran-Derivate, ein Verfahren zu ihrer Herstellung und ihre Verwendung**
Thienopyran derivatives, a process for their preparation, and their use
Dérivés de thiénopyrannes, un procédé pour leur préparation et leur utilisation

(30) Priorität: 27.06.1989 AT 1573/89
(43) Veröffentlichungstag der Anmeldung: 02.01.1991
(73) Patentinhaber: Chemisch Pharmazeutische Forschungsgesellschaft m.b.H., 4021 Linz (AT)
(72) Erfinder: Binder, Dieter, Dr., A-1190 Wien (AT); Rovenszky, Franz, Dr.Dipl.-Ing., A-2460 Bruck a.d. Leitha (AT); Weinberger, Josef, Dipl.-Ing., A-4540 Bad Hall (AT); Ferber, Hubert Peter, Dr., A-4021 Ansfelden (AT)

(56) Entgegenhaltungen:
- EP-A- 0 076 075
- EP-A- 0 120 428
- EP-A- 0 205 292
- EP-A- 0 277 611
- EP-A- 0 360 621
- WO-A-87/07607
- JOURNAL OF MEDICINAL CHEMISTRY, Band 29, 1986, Seiten 2194-2201; V.A. ASHWOOD et al.: "Synthesis and antihypertensive activity of 4-(cyclic amido)-2H-1-benzopyrans"
- ARZNEIMITTELCHEMIE I, E. Schroeder et al., Seiten 24-38, 1976, Georg Thieme Verlag, Stuttgart, DE

## Beschreibung

Die Erfindung betrifft neue Thienopyran-Derivate, ein Verfahren zu ihrer Herstellung und ihre Verwendung in Medikamenten zur Aktivierung von Membran-K⁺-Kanälen der glatten Muskulatur.

Aus der EP-A 0 076 075 sind in 4-Stellung durch N-Cycloalkanone substituierte Benzopyrane bekannt, die blutdrucksenkend wirken.

Es wurde nun gefunden daß Thienopyran-Derivate eine gegenüber den Substanzen der EP-A 0 076 075 verbesserte pharmakologische Wirkung besitzen.

Gegenstand der Erfindung sind daher neue Thienopyran-Derivate der allgemeinen Formel 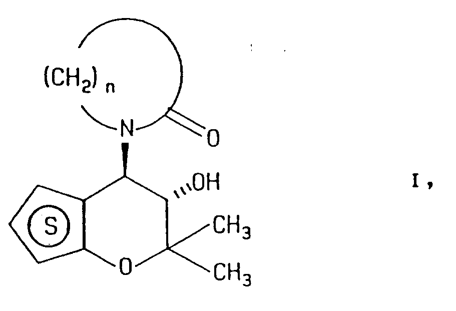
in der
der Rest 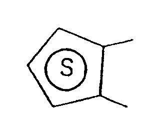
eine der Formeln 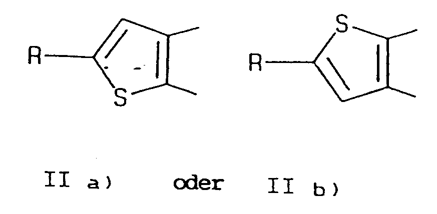
R -CN,
und
n eine ganze Zahl 3 bedeutet, ein Verfahren zu ihrer Herstellung, pharmazeutische Präparate, die diese Verbindungen enthalten sowie ihre Verwendung in Medikamenten zur Behandlung von Bluthochdruck und Asthma.

Die Verbindungen der Formel I sind chiral. Die vorliegende Erfindung umfaßt sowohl die Racemate der Verbindungen der Formel I als auch ihre Enantiomeren.

Eine besonders bevorzugte Einzelverbindungen ist:
6,7-Dihydro-5,5-dimethyl-6-hydroxy-trans-7-(2-oxo-1-pyrrolidinyl)-5H-thieno[3,2-b] pyran-2-carbonitril
Die Verbindungen der allgemeinen Formel I werden dadurch hergestellt, daß man eine Verbindung der allgemeinen Formel 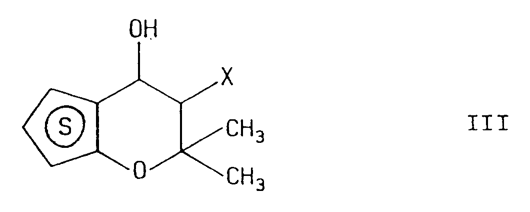
in der
der Rest 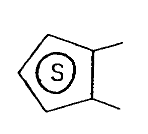
eine der Formeln 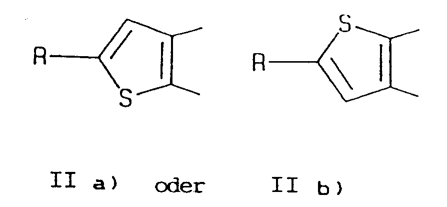
und X Chlor, Brom oder Jod bedeutet, mit einer Verbindung der allgemeinen Formel 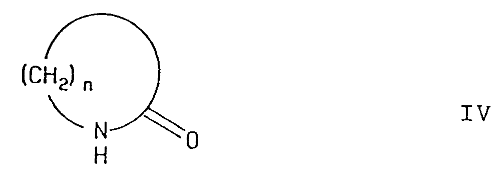
in der n die obige Bedeutung hat, in einem inerten organischen Verdünnungsmittel in Gegenwart von mindestens 2 Äquivalenten einer starken, nicht nucleophilen Base umsetzt und gegebenefalls das so erhaltene Racemat in die Enantiomeren trennt.

Die Umsetzung einer Verbindung der Formel III mit einer Verbindung der Formel IV erfolgt in einem reaktionsinerten, wasserfreien organischen Verdünnungsmittel, wie beispielsweise in DMF oder DMSO. Die Menge des verwendeten Verdünnungsmittels ist hierbei nicht kritisch, wenn ein absolut wasserfreies Verdünnungsmittel verwendet wird. Falls jedoch auch nur Spuren von Wasser im Verdünnungsmittel zurückgeblieben sind, sollte die verwendete Verdünnungsmittelmenge so gering wie nur möglich gehalten werden, doch muß andererseits darauf geachtet werden, daß noch für eine gute Durchmischung der Reaktionspartner beim Rühren gesorgt ist.

Zur Durchführung der Reaktion wird eine Verbindung der allgemeinen Formel III im Verdünnungsmittel aufgelöst und mit 1 - 3 Äquivalenten, bevorzugt mit einem geringen Überschuß einer starken, nicht nucleophilen Base wie z.B. Alkalihydrid oder Alkalitrimethylsilanolat unter Rühren versetzt. Die Base ist hierbei entweder im gleichen Verdünnungsmittel oder, im Fall des Alkalihydrids, in Paraffinöl gelöst Die Reaktionstemperatur liegt zwischen 0 und 40 °C, wobei Raumtemperatur bevorzugt ist. Es wird 5 Minuten bis 1 Stunde bis zur Beendigung der Epoxidbildung weitergerührt.

Anschließend werden nacheinander unter Rühren 1 - 3 Äquivalente, bevorzugt 1,2 bis 1,6 Äquivalente einer Verbindung der allgemeinen Formel IV, eventuell gelöst im gleichen Verdünnungsmittel, und mindestens ein weiteres Äquivalent, bevorzugt 1,2 bis 1,6 Äquivalente der obigen Base zugegeben. Ein größerer Überschuß der Base ist nicht schädlich; aus praktischen Gründen wird man jedoch trachten, daß die Gesamtmenge der Base 4 Äquivalente nicht übersteigt Die Reaktionstemperatur liegt zwischen 0 und 50 °C, bevorzugt bei Raumtemperatur. Die Dauer der Reaktion liegt zwischen 1 und 6 Stunden, wobei die längere Reaktionszeit den tieferen Temperaturen zuzuordnen ist.

Nach Beendigung der Reaktion wird die Reaktionslösung mit einer schwachen Säure, bevorzugt mit Eisessig, neutralisiert oder schwach sauer gestellt. Die weitere Aufarbeitung erfolgt nach üblichen und jedem Fachmann geläufigen Methoden wie beispielsweise Extraktion, Fällung oder Umkristallisation.

Bei obiger Umsetzung entsteht, falls nicht enantiomerenreine Ausgangsprodukte verwendet wurden, ein Racemat. Dieses kann nach üblichen und dem Fachmann geläufigen Methoden, z.B. analog J.M. Evens et.al., J.Med.Chem 29, 2194 (1986), getrennt werden. Man geht dabei beispielsweise so vor, daß man mit enantiomerenreinem alpha-Methylbenzylisocyanat ein Diastereomerenpaar eines Urethans erzeugt, dieses durch Kristallisation oder Säulenchromatographie trennt, und die Urethangruppe mit Trichlorsilan in Gegenwart von Triethylamin reduktiv wieder entfernt.

Die Verbindungen der allgemeinen Formel III können, ausgehend von Verbindungen der Formel V, gemäß dem folgenden Reaktionsschema und die Angaben in den Beispielen nach üblichen und dem Fachmann geläufigen chemischen Arbeitsmethoden hergestellt werden. 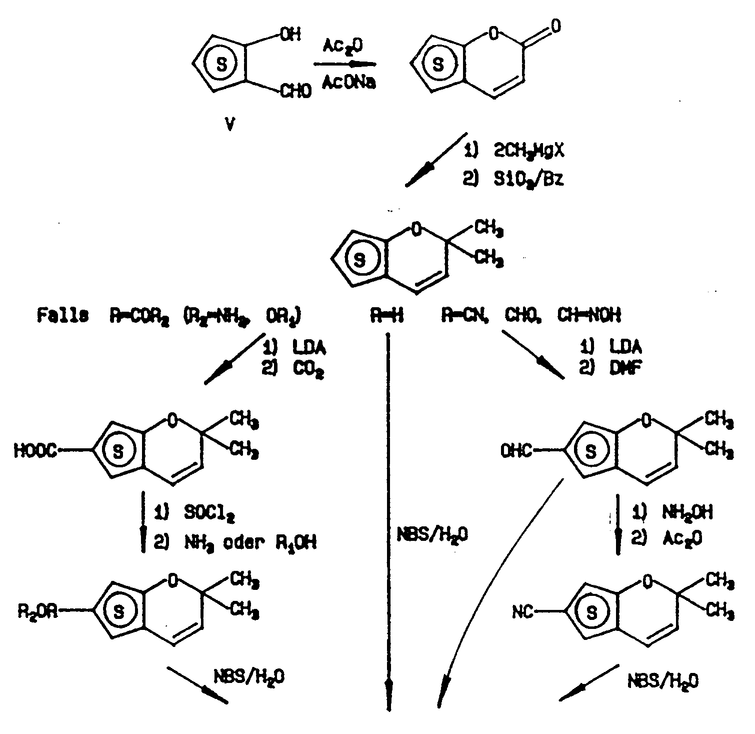
- LDA:: Lithiumdiisopropylamid
- DMF:: Dimethylformamid
- NBS:: N-Bromsuccinimid
Die Verbindungen der Formel IV sind literaturbekannt und käuflich erhältlich.

Die neuen Verbindungen der Formel I aktivieren in in vivo und in vitro - Modellen die Membran-K⁺-Kanäle der glatten Muskulatur.

Aufgrund dieser pharmakologischen Eigenschaften können die neuen Verbindungen allein oder in Mischung mit anderen Wirksubstanzen in Form üblicher galenischer Zubereitungen bei Erkrankungen, die durch Aktivierung von Membran-K⁺-Kanälen gelindert oder geheilt werden, wie z.B. Bluthochdruck oder Asthma, als Medikament verwendet werden.

Die Verbindungen der Formel I sind zur Verwendung am Menschen bestimmt und können auf übliche Weise, wie beispielsweise oral oder parenteral, verabreicht werden. Vorzugsweise werden sie oral verabreicht, wobei die Tagesdosis ca. 0,1 bis 100 mg/kg Körpergewicht beträgt, vorzugsweise 0,2 bis 20 mg/kg Körpergewicht. Der behandelnde Arzt kann jedoch, abhängig vom Allgemeinzustand und dem Alter des Papienten, der entsprechenden Substanz der Formel I, der Art der Krankheit und der Art der Formulierung, auch Dosen darüber oder darunter verschreiben.

Falls die erfindungsgemäßen Substanzen zur Prophylaxe verwendet werden, bewegen sich die Dosen ungefähr im selben Rahmen wie im Behandlungsfall. Die orale Verabreichung ist auch im Fall der Prophylaxe bevorzugt.

Die Verbindungen der Formel I können in Medikamenten allein oder in Verbindung mit anderen pharmazeutisch aktiven Substanzen verabreicht werden, wobei der Gehalt der Verbindungen der Formel I zwischen 0,1 und 99 % liegt. Im allgemeinen liegen die pharmazeutisch aktiven Verbindungen in einer Mischung mit geeigneten inerten Hilfs- und/oder Trägerstoffen oder Verdünnungsmitteln, wie z.B. pharmazeutisch unbedenklichen Lösungsmitteln, Gelatine, Gummi arabicum, Milchzucker, Stärke, Magnesiumstearat, Talk, pflanzlichen Ölen, Polyalkylenglykol, Vaseline und dergleichen vor.

Die pharmazeutischen Präparate können in fester Form, beispielsweise als Tabletten, Dragees, Suppositorien, Kapseln und dergleichen, in flüssiger Form, beispielsweise als Lösungen, Suspensionen oder Emulsionen oder in Zusammensetzungen mit verzögerter Freigabe des Wirkstoffes, vorliegen. Gegebenenfalls sind sie sterilisiert und enthalten Hilfsstoffe, wie Konservierungs-, Stabilisierungs- oder Emulgiermittel, Satze zur Veränderung des osmotischen Druckes und dergleichen.

Insbesondere können pharmazeutische Präparate die erfindungsgemäßen Verbindungen in Kombination mit anderen therapeutisch wertvollen Stoffen enthalten. Mit diesen können die erfindungsgemäßen Verbindungen beispielsweise zusammen mit den oben angegebenen Hilfs- und/oder Trägerstoffen oder Verdünnungsmitteln zu Kombinationspräparaten formuliert werden.

In den Beispielen verwendete Abkürzungen:
- **PE:**: Petrolether
- **EE:**: Essigsäureethylester
- **THF:**: Tetrahydrofuran
- **DMF:**: Dimethylformamid
- **DMSO:**: Dimethylsulfoxid

### Beispiel 1:

### trans-6,7-Dihydro-5,5-dimethyl-6-hydroxy-7-(2-oxo-1-pyrrolidinyl)5H-thieno[3,2-b] pyran-2-carbonitril

Zu 10,2 g (35,4 mMol) trans-6-Brom-6,7-dihydro-5,5-dimethyl-7-hydroxy-5H-thieno[3,2-b]pyran-2-carbonitril in 50 ml DMSO abs. werden 16,3 g (38,0 mMol) einer 26,2 %igen Lösung von Natriumtrimethylsilanolat in DMSO abs. bei Raumtemperatur zugetropft. Nach 20 Minuten Rühren werden 4,3 g (50,3 mMol) 2-Pyrrolidinon und anschließend 21,5 g (50,1 mMol) der Silanolatlösung zugetropft. Nach 2,5 Stunden werden 3,7 g (62,0 mMol) Eisessig zugetropft. Das Reaktionsgemisch wird zwischen 150 ml Wasser und 100 ml Ethylacetat verteilt, die Phasen getrennt und die wäßrige Phase mit 4 x 50 ml Ethylacetat extrahiert.
Die vereinten organischen Phasen werden mit Natriumsulfat/Aktivkohle getrocknet, filtriert und das Lösungsmittel abdestilliert. Es verbleiben 8,3 g einer orangebraunen, halbkristallinen Masse, welche mit Ethylacetat kristallisiert wird. Nach Abfiltrieren und Digerieren mit 2 x 15 ml eiskaltem Ethylacetat verbleibenden 5,0 g gelblicher Kristalle, die aus Ethanol/Aktivkohle umkristallisiert werden.
Ausbeute: 4,3 g farblose Kristalle (41,6 % d.Th.)
Fp: ab 233 °C Zers. (Ethanol)
Das Ausgangsprodukt kann wie folgt hergestellt werden:

### 5H-Thieno[3,2-b]pyran-5-on

95,0 g (0,75 Mol) 3-Hydroxy-thiophen-2-carbaldehyd, 380 g (3,71 Mol) Acetanhydrid und 61,0 g (0,74 Mol) wasserfreies Natriumacetat werden 88 Stunden zum Sieden erhitzt. Die Ölbadtemperatur wird während der Reaktion auf mindestens 170 °C gehalten. Das Gemisch wird zur Trockene eingedampft, die verbleibenden 240 g werden mit 500 ml Ether überschichtet und mit 600 ml gesättigter Kaliumhydrogencarbonatlösung neutralisiert Das Gemisch wird über Hyflo filtriert und der Filterkuchen mit 5 x 100 ml heißem Ethylacetat extrahiert. Die Phasen werden getrennt, die wäßrige Phase mit 3 x 200 ml Ethylacetat extrahiert. Die vereinten organischen Phasen werden mit 100 ml Wasser gewaschen.
Die organische Lösung wird mit Natriumsulfat/Aktivkohle getrocknet, filtriert und zu einem dicken Brei eingeengt, filtriert und der Filterkuchen mit 2 x 60 ml eiskaltem Ethylacetat digeriert. Die verbleibenden 74,0 g hellbraune Kristalle wrden aus Acetonitril/Aktivkohle umkristallisiert.
Ausbeute: 68,2g hellbeige Kristalle (60,5 % d.Th.)
Fp: 117 - 119 °C Zers. (Acetonitril)

### 5,5-Dimethyl-5H-thieno[3,2-b]pyran

Zu 370 ml (1,11 Mol) 3N Methylmagnesiumbromid in Ether wird eine Lösung von 65,0 g (0,43 Mol) 5H-Thieno[3,2-b]pyran-5-on in 650 ml THF abs. im Verlauf von ca. 35 Minuten so zugetropft, daß Rückfluß entsteht. Es wird 30 Minuten nachgerührt. Das Reaktionsgemisch wird auf eine Mischung aus 1125 %iger Ammoniumchloridlösung und 600 g Eis gegossen, die Phasen getrennt und die wäßrige Phase mit 3 x 200 ml Ether extrahiert. Die vereinten organischen Phasen werden mit Natriumsulfat/Aktivkohle getrocknet, filtriert und das Lösungsmittel abdestilliert. Die verbleibenden 98 g rotbraunes Öl werden in 600 ml Benzol abs. aufgenommen und mit 60g Kieselgel am Wasserabscheider 70 min zum Sieden erhitzt. Das Kieselgel wird abfiltriert, mit Methanol vollständig eluiert, das Filtrat mit Natriumsulfat getrocknet, filtriert und das Lösungsmittel abdestilliert. Die verbleibenden 74,5 g braunes Öl werden mit 60 ml Diisopropylether teilweise kristallisiert, filtriert, das Filtrat abermals eingedampft und der Rückstand (63 g) durch Säulenchromatographie (PE:EE = 9:1,350g Kieselgel KG 60) gereinigt.
Ausbeute: 34,3 g gelbe Kristalle (48,3 % d.Th.)
Fp: 28-32°C
Kp: 60-65°C/16,0 mbar

### 5,5-Dimethyl-5H-thieno[3,2-b]pyran-2-carbaldehyd

Zu 27,0g (0,27 Mol) Diisopropylamin in 100 ml abs. THF werden bei -40°C 96 ml (0,24 Mol) 2,5 N n-Butyllithium in n-Hexan getropft. Diese Lösung wird bei -40 °C mit 29,1 g (0,18 Mol) 5,5-Dimethyl-5H-thieno[3,2-b]pyran im 330 ml THF abs. versetzt und das Gemisch bei Stunden bei 0 °C gehalten Die dunkelrote Lösung wird bei -50 °C mit 19,5 g (0,27 Mol) DMF in 80 ml THF abs. umgesetzt. Das Reaktionsgemisch wird an Raumtemperatur angleichen gelassen, auf 11 Eiswasser gegossen und mit 2 N Salzsäure auf pH 4 gestellt. Die Phasen werden getrennt, die wäßrige Phase mit 3 x 100 ml Ether extrahiert, die vereinten organischen Phasen mit 100 ml Wasser gewaschen, mit Natriumsulfat/Aktivkohle getrocknet, filtriert und das Lösungsmittel abdestilliert.
Die verbleibenden 45 g dunkelbraunen Öles werden durch Säulenchromatographie gereinigt (PE:EE = 8:1,200g KG 60).
Das erhaltene orange Öl wird mit Diisopropylether Kristallisiert, filtriert und mit 2 x 35 ml eiskaltem Diisopropylether digeriert.
Ausbeute: 23,5 g gelbe Kristalle (69,1 % d. Th.)
Fp: 58 - 60 °C Zers. (Diisopropylether)

### 5,5-Dimethyl-5H-thieno[3,2-b]pyran-2-carbonitril

Zu 9,0g (138 mMol) Hydroxylamin-hydrochlorid in 70 ml Methanol abs. und 7,74 g (138 mMol) Kaliumhydroxid werden 18,45 g (95 mMol) 5,5-Dimethyl-5H-thieno[3,2-b]pyran-2-carbaldehyd gegeben, wobei sich ein pH-Wert von 7 einsteilt. Nach 90 min Rühren bei Raumtemperatur wird bei 30 °C das Lösungsmittel abgezogen und der Rücktstand zwischen 100 ml Wasser und 100 ml Methylenchlorid verteilt. Die Phasen werden getrennt, die wäßrige Phase mit 3 x 60 ml Methylenchlorid extrahiert, die vereinten organischen Phasen mit 40 ml Wasser gewaschen, mit Natriumsulfat getrocknet, filtriert und das Lösungsmittel abdestilliert.

Es verbleiben 26,0 g Rückstand,welcher in 80 ml Acetanhydrid 10 min zum Sieden erhitzt wird. Das Lösungsmittel wird im Vakuum abgezogen und der Rückstand mit 70 ml Ether überschichtet. Die Suspension wird mit Natriumcarbonatlösung neutralisiert und über Hyflo filtriert. Die Phasen werden getrennt, die wäßrig Phase mit 3 x 30 ml Ether extrahiert, die vereinten organischen Phasen mit Natriumcarbonatlösung gewaschen, mit Natriumsulfat/Aktivkohle getrocknet, filtriert und das Lösungsmittel abdestilliert.
Ausbeute: 13,2g orangebraune Kristalle (72,5 % d. Th.)
Fp: 73 - 75 °C (Petrolether)

### trans-6-Brom-6,7-dihydro-5,5-dimethyl-7-hydroxy-5H-thieno[3,2-b]-pyran-2-carbonitril

Zu 11,0 g (57,5 mMol) 5,5-Dimethyl-5H-thieno[3,2-b]pyran-2-carbonitril in 100 ml DMSO abs. werden 2,1 g (117 mMol) Wasser und 20,5 g (115 mMol) N-Bromsuccinimid gegeben.
Nach 20 min. Rühren wird das Reaktionsgemisch zwischen 250 ml Wasser und 150 ml Ethylacetat verteilt, die wäßrige Phase mit Natriumchlorid gesättigt. Die Phasen werden getrennt. Die wäßrige Phase wird mit 3 x 50 ml Ethylacetat extrahiert. Die vereinten organischen Phasen werden mit 2 x 70 ml gesättigter Kaliumhydrogencarbonatlösung und 50 ml Wasser gewaschen. Die organische Lösung wird mit Natriumsulfat/Aktivkohle getrocknet, filtriert und das Lösungsmittel abdestilliert. Die verbleibender 18,0 g hellgelber Kristalle werden aus Methanol mit Aktivkohle umkristallisiert.
Ausbeute: 14,2 g farblose Kristalle (85,7 % d.Th.)
Fp: 155,5 - 156,0 °C Zers. (Methanol)

### Beispiel 2

### Racemattrennung

Das Schema der Racemattrennung ist im folgenden Formelschema dargelegt:

### Formelschema Racemattrennung

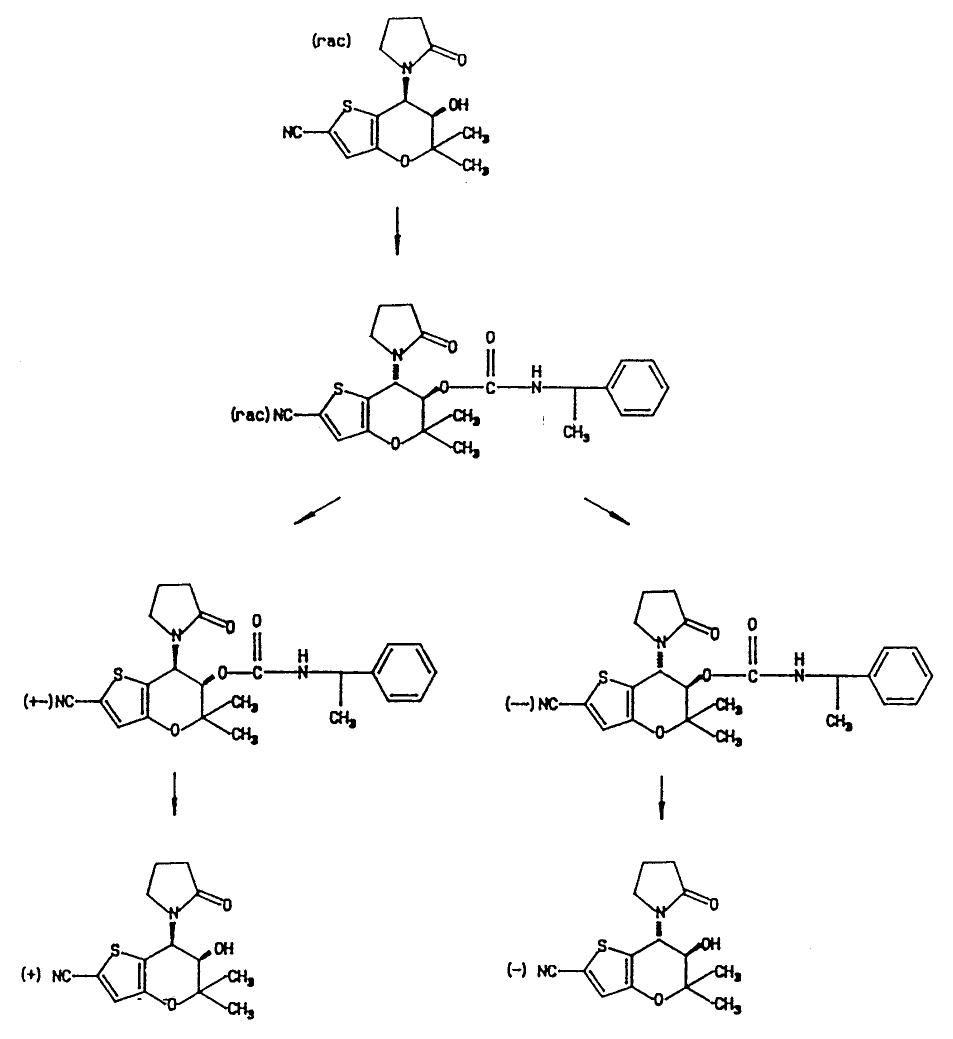

### 1. Umsetzung zu rac-2-Cyano-6,7-dihdro-7-(2-oxo-1-pyrrolidinyl)-N-[(1-phenyl)-ethyl]-5H-thieno[3,2-b]pyran-6-carbamat

4,5 g (15,4 mMol) rac-6,7-Dihydro-5,5-dimethyl-6-hydroxy-7-(2-oxo-1-pyrrolidinyl)-5H-thieno[3,2-b]pyran-2-carbonitril werden mit 2,49 g (16,9 mMol (S)-(-)-alpha-Methylbenzylisocyanat in Toluol suspendiert und zum Sieden erhitzt. Nach 70 Stunden ist die Reaktion vollständig und die nunmehr klare Lösung wird zur Trockene eingedampft.
Die verbleibenden 6,85 g gelblicher Kristalle werden aus Aceton und Aktivkohle umkristallisiert.
Ausbeute: 5,31 g farblose Kristalle (78,5 % d. Th.)
Fp: 203 - 207 °C (Aceton)

### 2. Trennung der Diastereomeren

Die Trennung erfolgt durch Säulenchromatographie, wobei die erhaltenen Mischfraktionen jeweils wieder getrennt werden.
Laufmittel: CHCl₃:ET₂O = 5:1
Stationäre Phase: 300g Kieselgel KG 60
Ausgangsmenge: 5,3 g Diastereomerengemisch

### Ergebnis aus 3 Trennungen:

**Tabelle 1**

| | Erste | Zweite Trennung | Dritte | Summe |
|---|---|---|---|---|
| 1. Fraktion | 0,82 g | 0,68 g | 0,29 g | 1,79 g |
| Mischfraktion | 3,80g | 2,35 g | 1,61 g | 1,61 g |
| 2. Fraktion | 0,52g | 0,56 g | 0,59 g | 1,67 g |

### a) 1. Fraktion:

(+ -)-2-Cyano-6,7-dihydro-7-(2-oxo-1-pyrrolidinyl)-N-[(1-phenyl)-ethyl]-5H-thieno (3,2-b]pyran-6-carbamat
Fp: 198 - 200 °C (Aceton)
^{c}1(Aceton) - 0,52°

### b) 2. Fraktion:

(--)-6-Cyano-6,7-dihydro-7-(2-oxo-1-pyrrolidinyl)-N-[(1-phenyl)-ethyl]-5H-thieno [3,2b]pyran-6-carbamat
Fp: 203 - 205 °C (Aceton)
^{c}1(Aceton) - 0,44°

### 3. Abspaltung der Hilfsgruppe

Jeweils 1,4 g (3,2 mMol) getrenntes Diastereomer wird unter Stickstoffatmosphäre in 12 ml Toluol mit 0,65 g (6,4 mMol) Triethylamin gelöst. Bei 40 °C wird 0,87 g (6,4 mMol) Trichlorsilan zugetropft und 3 Stunden bei dieser Temperatur gerührt. Es wird über Nacht bei Raumtemperatur nachgerührt.
Das Reaktionsgemisch wird mit 1,5 ml Methanol versetzt und 15 min auf 80 °C gehalten, abkühlen gelassen und das ausgefallene Ammoniumchlorid abfiltriert.
Nach dem Abziehen des Lösungsmittels wird das Rohprodukt einer chromatographischen Reinigung unterworfen (70 g Kieselgel KG 60, Eluens: Ethylacetat).
Das erhaltene Produkt wird nochmals aus Ethanol umkristallisiert.

### a) (+)trans-6,7-Dihydro-5,5-dimethyl-6-hydroxy-7-(2-oxo-1-pyrrolidinyl)-5H-thieno[3,2-b]pyran-2-carbonitril

Ausbeute: 0,66 farblose Kristalle (71,0 % d. Th.)
^{c}1(Methanol) + 0,93°
Fp: 187 °C Zers. (Ethanol)

### b) (-)trans-6,7-Dihydro-5,5-dimethyl-6-hydrory-7-(2-oxo-1-pyrrolidinyl)-5H-thieno [3,2-b]pyran-2-carbonitril

Ausbeute: 0,74 g farblose Kristalle (79,6 % d.Th.)
^{c}1 (Methanol) - 0,91°
Fp: 189 °C Zers. (Ethanol)

### Beispiel 3

### Wirkungen der Substanz des Beispiels 1 auf den Blutdruck der wachen, spontan hypertensiven Ratte

Die Untersuchungen wurden an spontan hypertensiven Ratten (Fa. Madörin, Schweiz, 245 - 395 g) durchgeführt. Die Versuchstiere hatten einen Katheter in der die A. carotis dauernd eingebunden, der zur Blutdruckmessung mit einem Transducer (Fa. Statham) verbunden war. Die Daten wurden in einem Hellige Cardiovascular Analyser aufbereitet und auf einem Watanabe WR 3101-4-Schreiber aufgezeichnet.

Die Verbindung des Beispiels 1 wurde in einer 10 %igen alkoholischen Lösung aufgelöst und entweder intravenös in die Schwanzvene oder oral verabreicht. Als Kontrolle wurde das Lösungsmittel ohne Testsubstanz verabreicht. Die Wirkungen auf den Blutdruck wurden bis eine Stunde nach der i.v.-Verabreichung und bis 3 Stunden nach der oralen Gabe der Substanz aufgezeichnet.

Pro Konzentration wurden 4 - 5 Versuche durchgeführt. Aus den so erhaltenen Werten wurde durch lineare Regressionsanalyse die ED₂₀ (der Wert, der den diastolischen Blutdruck um 20 % reduziert) errechnet.
Die Ergebnisse sind in Tabelle 2 zusammengefaßt.

**Tabelle 2**

| Konz. (mcg/kg) | Änderung des systolischen Blutdrucks in % | Änderung des diastolischen Blutdrucks in % |
|---|---|---|
| 0 (Kontr.) | - 1,8 % (± 2,5) | - 1,5 % (± 4,4) |
| 10 (i.v.) | -9,2 % (± 3,4) | -5,6 % (± 7,3) |
| 30 (i.v.) | - 30,0 % (± 4,8) | -46,0 % (± 6,0) |
| 100 (i.v.) | -41,0 % (± 6,6) | - 65,5 % (± 3,7) |
| 30 (p.o.) | - 2,3 % (± 2,5) | - 4,5 % (± 2,9) |
| 100 (p.o.) | - 26,5 % (± 5,4) | - 23,0 % (± 6,7) |
| 300 (p.o.) | - 38,0 % (± 12,2) | - 36,8 % (± 13,1) |

Aus diesen Werten errechnet sich eine ED₂₀ i.v. von 15,5 mcg/kg und eine ED₂₀ p.o. von 90,4 mcg/kg.

### Beispiel 4

Beispiel 4 wurde mit der Ausnahme wiederholt, daß als Testsubstanz Cromakalim (3,4-Dihydro-2,2-dimethyl-3-hydroxy-trans-4-(2-oxo-1-pyrrolidinyl)2H-benzo[b]pyran-6-carbonitril, die bevorzugte Substanz der EP-A 0 076 075) verwendet wurde. Die Ergebnisse dieser Versuche sind in Tabelle 3 zusammengefaßt.

**Tabelle 3**

| Konz (mcg/kg) | Änderung des systolischen Blutdrucks in % | Änderung des diastolischen Blutdrucks in % |
|---|---|---|
| 0 (Kontr.) | - 1,8 % (± 2,5) | - 1,5 % (± 4,4) |
| 10 (i.v.) | - 6,6 % (± 4,0) | - 6,6 % (± 6,3) |
| 30 (i.v.) | -26,5 % (± 5,9) | - 27,5 % (± 7,9) |
| 100 (i.v.) | -36,3 % (± 9,0) | - 58,3 % (± 7,9) |
| 30 (p.o.) | -3,3 % (± 1.0) | - 4,3 % (± 1,5) |
| 100 (p.o.) | -11,5 % (± 6,8) | -11,3 % (± 9,2) |
| 300 (p.o.) | -22,8 % (± 10,8) | -24,5 % (± 11,3) |

Aus diesen Werten ergibt sich eine ED₂₀ i.v. von 19,4 mcg/kg und eine ED₂₀ p.o. von 213,4 mcg/kg.

## Patentansprüche

1. Thienopyrane der allgemeinen Formel 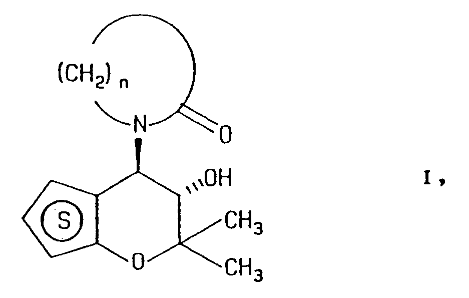 in der
der Rest 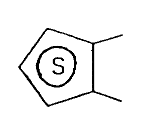 eine der Formeln 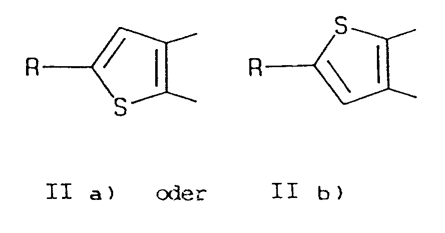 R -CN
und
n eine ganze Zahl 3, bedeutet.

2. Trans-6,7-Dihydro-5,5-dimethyl-6-hydroxy-7-(2-oxo-1-pyrrolidinyl)-5H-thieno[3,2-b] pyran-2-carbonitril.

3. Verbindungen der Formel I nach Anspruch 1 in enantiomerenreiner Form.

4. Verbindungen der Formel I nach Anspruch 1 in Mischungen der optischen Antipoden.

5. Verfahren zur Herstellung von Verbindungen der allgemeinen Formel I nach Anspruch 1, dadurch gekennzeichnet, daß man eine Verbindung der allgemeinen Formel 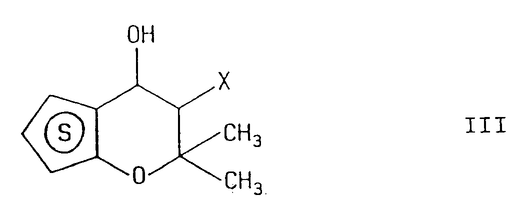 in der
der Rest 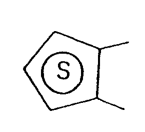 eine der Formeln 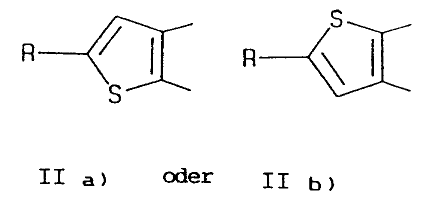 und X Chlor, Brom oder Jod bedeutet, mit einer Verbindung der allgemeinen Formel 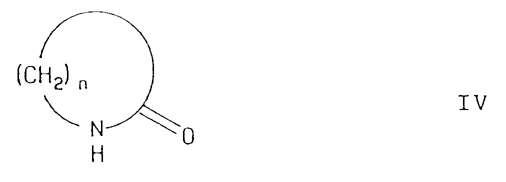 in der n eine ganze Zahl 3, bedeutet, in einem inerten organischen Verdünnungsmittel in Gegenwart von mindestens 2 Äquivalenten einer starken, nicht nucleophilen Base umsetzt und gegebenenfalls das so erhaltene Racemat in die Enantiomeren trennt.

6. Pharmazeutische Präparate, enthaltend Verbindungen der allgemeinen Formel I nach Anspruch 1 in Kombination mit üblichen galenischen Hilfs- und/oder Trägerstoffen oder Verdünnungsmitteln.

7. Pharmazeutische Präparate, enthaltend Verbindungen der allgemeinen Formel I nach Anspruch 1 in Kombination mit anderen therapeutisch wertvollen Wirkstoffen sowie üblichen galenischen Hilfs- und/oder Trägerstoffen oder Verdünnungsmitteln.

8. Verbindungen der Formel I nach Anspruch 1 zur Verwendung als Wirkstoffe für Arzneimittel zur Behandlung von Krankheiten, die durch Aktivierung von Membran-K⁺-Kanälen gelindert oder geheilt werden können.

## Claims

1. Thienopyrans of the general formula 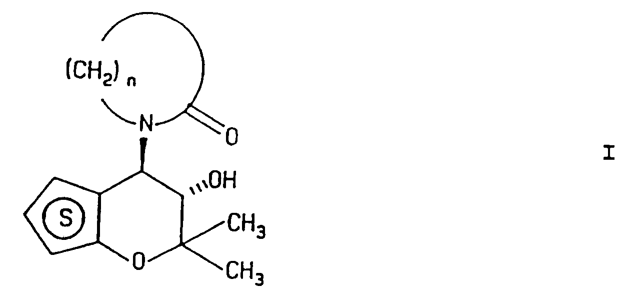 in which
the radical 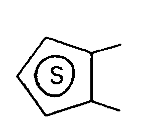 denotes one of the formulae 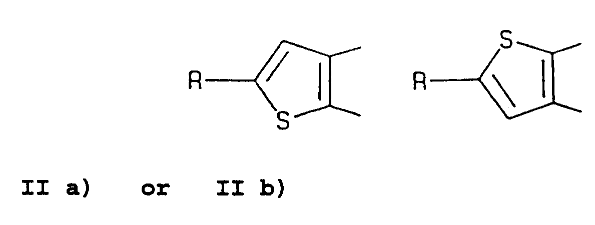 R denotes -CN
and
n denotes an integer 3.

2. trans-6,7-Dihydro-5,5-dimethyl-6-hydroxy-7-(2-oxo-1-pyrrolidinyl)-5H-thieno[3,2-b]pyran-2-carbonitrile.

3. Compounds of the formula I according to Claim 1 in enantiomerically pure form.

4. Compounds of the formula I according to Claim 1 in mixtures of the optical antipodes.

5. Process for the preparation of compounds of the general formula I according to Claim 1, characterized in that a compound of the general formula 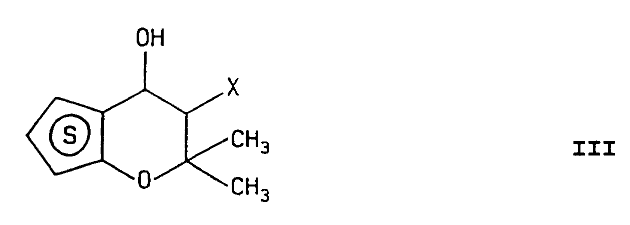 in which
the radical 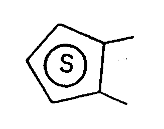 denotes one of the formulae 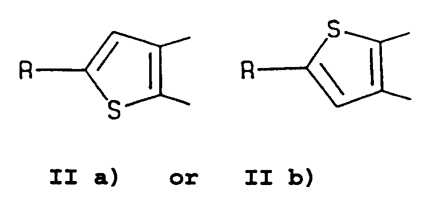 and X denotes chlorine, bromine or iodine, is reacted with a compound of the general formula 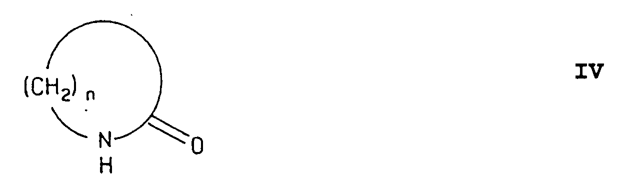 in which n denotes an integer 3, in an inert organic diluent in the presence of at least 2 equivalents of a strong, non-nucleophilic base and, if appropriate, the racemate thus obtained is separated into the enantiomers.

6. Pharmaceutical preparations containing compounds of the general formula I according to Claim 1 in combination with customary pharmaceutical auxiliaries and/or excipients or diluents.

7. Pharmaceutical preparations containing compounds of the general formula I according to Claim 1 in combination with other therapeutically useful active compounds and customary pharmaceutical auxiliaries and/or excipients or diluents.

8. Compounds of the formula I according to Claim 1 for use as active compounds for medicaments for the treatment of diseases which can be alleviated or cured by activation of membrane K⁺ channels.

## Revendications

1. Thiénopyranes de la formule générale 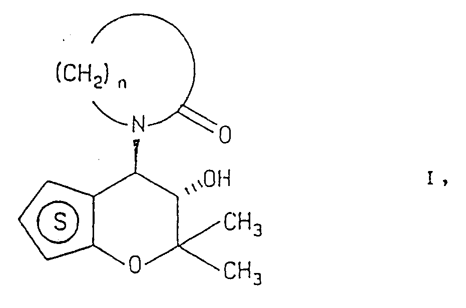 dans laquelle
le groupe 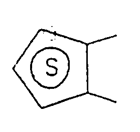 représente l'une des formules 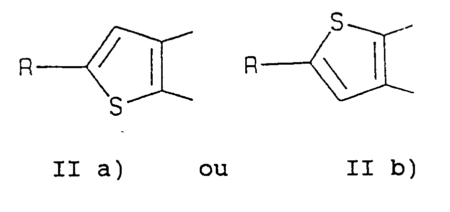 R -CN
et
n un chiffre entier 3.

2. trans-6,7-dihydro-5,5-diméthyl-6-hydroxy-7-(2-oxo-1-pyrrolidinyl)5H-thieno[3,2-b]-pyrane-2-carbonitrile.

3. Combinaisons de la formule I selon revendication 1, dans une forme purement énantiomère.

4. Combinaisons de la formule I selon revendication 1 dans des mélanges des antipodes optiques.

5. Procédé de fabrication de combinaisons de la formule générale I selon revendication 1, caractérisé par le fait que l'on transpose une combinaison de la formule générale 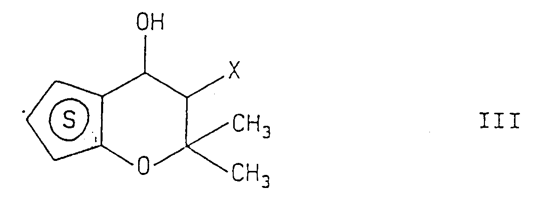 dans laquelle le groupe 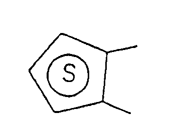 représente l'une des formules 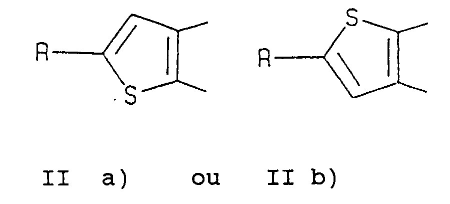 et X du chlore, du brome ou de l'iode, avec une combinaison de la formule générale 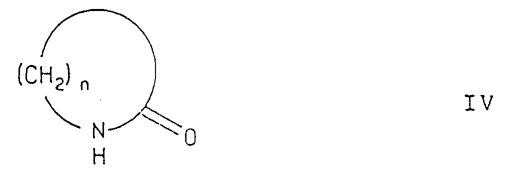 dans laquelle n signifie un chiffre entier 3, dans un diluant inerte organique, en présence d'au moins 2 équivalents d'une forte base non-nucléophile et que l'on sépare, le cas-échéant, le racémate ainsi obtenu dans les énantiomères.

6. Préparations pharmaceutiques contenant des combinaisons de la formule générale I selon revendication 1 en combinaison avec les substances galéniques auxiliaires et/ou de support usuelles ou des diluants.

7. Préparations pharmaceutiques contenant des combinaisons de la formule générale I selon revendication 1 en combinaison avec d'autres substances à activité thérapeutique ou des substances galéniques auxiliaires et/ou de support usuelles ou des diluants.

8. Combinaisons de la formule I selon revendication 1 pour utilisation en tant que principe actif dans des médicaments destinés au traitement de maladies pouvant être allégées ou guéries par activation des canaux de membrane K+.
